(19) Europäisches Patentamt
European Patent Office
Office européen des brevets

(11) **EP 3 445 455 B1**

(12) **EUROPÄISCHE PATENTSCHRIFT**

(45) Veröffentlichungstag und Bekanntmachung des Hinweises auf die Patenterteilung:
**24.02.2021 Patentblatt 2021/08**

(21) Anmeldenummer: **17715414.3**

(22) Anmeldetag: **24.03.2017**

(51) Int Cl.:
***A61Q 17/04*** *(2006.01)*    ***A61B 5/103*** *(2006.01)*
***A61K 8/30*** *(2006.01)*    *A61B 5/00 (2006.01)*

(86) Internationale Anmeldenummer:
**PCT/EP2017/057058**

(87) Internationale Veröffentlichungsnummer:
**WO 2017/182232 (26.10.2017 Gazette 2017/43)**

(54) **VERFAHREN ZUR BESTIMMUNG DES UV-SCHUTZES VON SONNENSCHUTZMITTELN AUF DER HAUT UNTER WASSER**

METHOD FOR DETERMINING THE UV PROTECTION OF SUNCREEN ON THE SKIN WHEN UNDER WATER

PROCÉDÉ PERMETTANT DE DÉTERMINER LA PROTECTION UV OFFERTE PAR DES PRODUITS DE PROTECTION SOLAIRE À LA PEAU SOUS L'EAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.04.2016 DE 102016206466**

(43) Veröffentlichungstag der Anmeldung:
**27.02.2019 Patentblatt 2019/09**

(73) Patentinhaber: **Beiersdorf AG
20253 Hamburg (DE)**

(72) Erfinder:
• **BATZER, Jan
22459 Hamburg (DE)**
• **WEISE, Julia Maxi
22529 Hamburg (DE)**
• **SCHLÄGER, Torsten
22297 Hamburg (DE)**
• **BLECKMANN, Andreas
22926 Ahrensburg (DE)**
• **LESSMANN, Michael
21423 Winsen-Luhe (DE)**

(56) Entgegenhaltungen:
WO-A1-98/55086    DE-A1- 10 214 054

• **Anonymous: "GNPD - SPF 50 Face Cream", , 1. August 2014 (2014-08-01), XP055283407, Gefunden im Internet: URL:http://www.gnpd.com/sinatra/recordpage /2595545/from_search/pTY9aSCViI/?page=1 [gefunden am 2016-06-24]**
• **MARGINEAN LAZAR G ET AL: "SUNSCREEN'S PHOTOCHEMICAL BEHAVIOUR: IN VIVO EVALUATION BY THE STRIPPING METHOD", INTERNATIONAL JOURNAL OF COSMETIC SCIENCE, KLUWER ACADEMIC PUBLISHERS, DORDRECHT, NL, Bd. 19, Nr. 2, 1. Januar 1997 (1997-01-01) , Seiten 87-101, XP001165609, ISSN: 0142-5463, DOI: 10.1046/J.1467-2494.1997.171703.X**
• **PANTINI G ET AL: "Protective and sunscreen emulsions containing a perfluoropolyether amide", SOFW-JOURNAL SEIFEN, OELE, FETTE, WACHSE, VERLAG FUR CHEMISCHE INDUSTRIE, AUGSBURG, DE, Bd. 127, Nr. 5, 1. Mai 2001 (2001-05-01), Seiten 13-17, XP001206575, ISSN: 0942-7694**

**Beschreibung**

Die vorliegende Erfindung betrifft ein Verfahren zur Bestimmung des in-vivo UV-Lichtschutzes (SPF) von Sonnenschutzmitteln auf der Haut im Wasser bzw. zum Nachweis des Schutzes vor UV-Strahlung unter Wasser.

Der Trend weg von der vornehmen Blässe hin zur "gesunden, sportlich braunen Haut" ist seit Jahren ungebrochen. Um diese zu erzielen, setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko, an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Insbesondere erweist es sich als schwierig, den UV-Schutz eines Sonnenschutzmittels während des Badens (beispielsweise im Schwimmbad, in Badeseen oder im Meerwasser) für die Haut zu bestimmen bzw. vorherzusagen. Da Wasser eine nur geringe UV-Absorption aufweist, kann es insbesondere bei längerem Aufenthalt im Wasser zu UV-Strahlungsbedingten Schäden der Haut (insbesondere bei nicht vorgebräunten, üblicherweise durch Kleidungsstücke von Sonnenlicht geschützten Hautpartien) kommen. Um diesbezüglich böse Überraschungen (insbesondere in Form von Sonnenbrand) zu vermeiden, war es die Aufgabe der vorliegenden Erfindung, ein reproduzierbares Verfahren zu entwickeln, mit dem der in-vivo Schutz der Haut durch ein Sonnenschutzmittel beim Baden vorhersagbar wird. Überraschend gelöst wird die Aufgabe durch ein Verfahren zur Bestimmung des in-vivo UV-Lichtschutzes (SPF) von Sonnenschutzmitteln auf der Haut im Wasser gemäß Anspruch 1. Zwar kennt der Fachmann Bestimmungsmethoden zur Ermittlung des Lichtschutzfaktors (SPF) wie die ISO-Norm ISO24444 der International Standards Organisation, Genf (2010), der Cosmetics Europe (International Sun Protecting Factor (SPF) Test Method (2006)) oder den Guidlines for Evaluation Sun Product Water Resistence (Cosmetics Europe Guidelines 2005), doch konnten diese Offenbarungen nicht den Weg zur vorliegenden Erfindung weisen, da sie sich auf den Schutz der Haut an der Luft vor oder nach einem Bad beziehen. Insbesondere temporäre Effekte im Wasser, wie beispielsweise das Phasenverhältnis von Öl- und Wasserphase einer Emulsion, bleiben bei diesen Methoden unberücksichtigt.

DE10214054 A1 offenbart ein Verfahren zur Bestimmung des Lichtschutzes eines Sonnenschutzmittels. Das Sonnenschutzmittel wird auf Hautareale aufgetragen und nach dem Einwirken wird die Haut mit Wasser in Kontakt gebracht. Anschließend erfolgt die Bestimmung des Lichtschutzfaktors.

Marginean Lazar et al, Sunscreens' photochemical behaviour: in vivo evaluation by the stripping method, International Journal of Cosmetic Science, Kluwer Academic Publishers, Dordrecht, NL, Bd. 19, Nr. 2, 1. Januar 1997, Seiten 87-101, ISSN: 0142-5463, offenbart ein Verfahren zur Bestimmung des Lichtschutzes bei dem die Strahlungsquelle mit einem Wasserfilter versehen ist.

WO98/55086 A1 offenbart ein Verfahren zur Bestimmung des Lichtschutzes bei dem das Sonnenschutzmittel unter Wasser aufgetragen wird.

Pantini et al., Protective and sunscreen emulsions containing a perfluoropolyether amide, SOFW-JOURNAL, SEIFEN, OELE, FETTE, WACHSE, VERLAG FUR CHEMISCHE INDUSTRIE, AUGSBURG, DE, Bd. 127, Nr. 5, 1. Mai 2001, Seiten 13-17, ISSN: 0942-7694, offenbart ein Verfahren zur Bestimmung der Wasserfestigkeit von Sonnenschutzmitteln, bei dem der Arm mit dem Sonnenschutzmittel in einem Wasserbad bewegt wird. Neben der Bestimmung des in-vivo UV-Lichtschutzes (SPF) von Sonnenschutzmitteln auf der Haut im Wasser dient das erfindungsgemäße Verfahren erfindungsgemäß auch generell zum Nachweis des Schutzes vor UV-Strahlung unter Wasser.

Als erfindungsgemäßes Hautareal der Testperson können grundsätzlich alle Hautareale eingesetzt werden, beispielsweise Areale auf dem Unterarm, der Wade oder dem Rücken. Erfindungsgemäß bevorzugt ist es, wenn als Hautareal ein Areal des Unterarms ausgewählt wird.

So ist es erfindungsgemäß vorteilhaft, wenn im Verfahrensschritt a) das definierte Areal als Teil des Unterarms einer Testperson, welches mit einer definierten Menge an zu testendem Sonnenschutzmittel bestrichen wird, die Größe von 12 cm$^2$ bis 64 cm$^2$ aufweist. Erfindungsgemäß bevorzugt hat das Areal dabei eine Größe von 35 cm$^2$ cm.

Außerdem ist es erfindungsgemäß von Vorteil, wenn man im Verfahrensschritt a) das Sonnenschutzmittel mit Hilfe eines Fingerlings auf die Haut aufträgt.

Vorteilhaft im Sinne der vorliegenden Erfindung ist es auch, wenn man im Verfahrensschritt a) das Sonnenschutzmittel in einer Menge von 2 mg/cm$^2$ auf die Haut aufträgt, wobei diese Auftragungsmenge üblicherweise einen Toleranzbereich von 1 Gewichts-% aufweist.

Es ist erfindungsgemäß von Vorteil, wenn man im Verfahrensschritt b) das Sonnenschutzmittel über einen Zeitraum von 15 Minuten in die Haut einziehen lässt. Hierbei beträgt der Toleranzbereich üblicherweise 1 Minute.

Vorteilhaft im Sinne der vorliegenden Erfindung ist es, wenn die UV-Licht undurchlässige Schablone, die in Verfahrensschritt c) auf den Unterarm aufgebracht wird, eine den Unterarm umschließende Arm-Manschette ist.

Ferner ist es vorteilhaft im Sinne der vorliegenden Erfindung, wenn die UV-Licht undurchlässige Schablone, die in Verfahrensschritt c) auf den Unterarm aufgebracht wird, aus Baumwollgewebe mit einer Acrylatbeschichtung besteht.

Erfindungsgemäß vorteilhafte Ausführungsformen der vorliegenden Erfindung sind dadurch gekennzeichnet, dass die UV-Licht undurchlässige Schablone, die in Verfahrensschritt c) auf den Unterarm aufgebracht wird, 8-20 Öffnungen definierter Größe aufweist, wobei die Anzahl von 12 Öffnungen definierter Größe erfindungsgemäß bevorzugt ist.

Darüber hinaus ist es erfindungsgemäß von Vorteil, wenn die UV-Licht undurchlässige Schablone, die in Verfahrensschritt c) auf den Unterarm aufgebracht wird, kreisförmige Öffnungen aufweist.

Vorteilhaft im Sinne der vorliegenden Erfindung ist es, wenn die UV-Licht undurchlässige Schablone, die in Verfahrensschritt c) auf den Unterarm aufgebracht wird, kreisförmige Öffnungen mit einem Durchmesser von jeweils 1 cm aufweist.

Erfindungsgemäß vorteilhaft ist es auch, wenn die UV-Licht undurchlässige Schablone, die in Verfahrensschritt c) auf den Unterarm aufgebracht wird, so fixiert wird, dass jeweils die gleiche Anzahl der Öffnungen über den mit dem Sonnenschutzmittel behandelten Teil des Unterarms und dem mit dem Sonnenschutzmittel unbehandelten Teil des Unterarms, liegt. Die Fixierung kann dabei beispielsweise mit Hilfe eines doppelseitig klebenden Klebebandes erfolgen. Auch können die Enden der Schablone mit Hilfe eines wasserfesten Klebebandes (z.B. Leukosilk®) befestigt werden.

Vorteilhaft im Sinne des erfindungsgemäßen Verfahrens ist es, wenn der mit der Schablone präparierte Unterarm in Verfahrensschritt d) in ein wasserdichtes Behältnis gelegt wird, welches eine Länge von 530mm, eine Breite von 375mm und eine Höhe von 200mm aufweist. Im Zweifelsfalle kann auch ein etwas größeres oder kleineres Behältnis gewählt werden. Entscheidend ist, dass der präparierte Unterarm genügend Platz im Behältnis hat und vollständig mit Wasser bedeckt werden kann.

Für das erfindungsgemäße Verfahren ist es ferner erfindungsgemäß von Vorteil, wenn das wasserdichte Behältnis aus Kunststoff besteht.

Erfindungsgemäß vorteilhaft für das erfindungsgemäße Verfahren ist es, dass der mit der Schablone präparierte Unterarm in Verfahrensschritt d) in ein wasserdichtes Behältnis gelegt wird, welches mit Wasser befüllt ist, das während der Verfahrensdurchführung eine konstante Temperatur aufweist. Dabei ist eine Schwankung der Wassertemperatur während der Durchführung von $\pm$ 2°C akzeptabel.

Vorteilhaft im Sinne der vorliegenden Erfindung ist es, wenn der mit der Schablone präparierte Unterarm in Verfahrensschritt d) in ein wasserdichtes Behältnis gelegt wird, welches mit Wasser befüllt ist, das während der Verfahrensdurchführung eine konstante Temperatur von 15 bis 35 °C aufweist. Um dem Probanden und seinem Unterarm die Messung möglichst angenehm zu machen, wird üblicherweise eine Wassertemperatur von 29 °C gewählt. Zu Normzwecken kann aber genauso gut auf eine Wassertemperatur von 20 °C oder 25 °C zurückgegriffen werden.

Es ist erfindungsgemäß von Vorteil, wenn der mit der Schablone präparierte Unterarm in Verfahrensschritt d) in ein wasserdichtes Behältnis gelegt wird, welches mit Wasser befüllt ist, einen Salzgehalt von 0 bis 30 Gewichts-% und einen Chlorgehalt von 0 bis 3 mg/l aufweist. Mit der Variation des Salz- bzw. Chlorgehaltes kann in erfindungsgemäß Leitungswasser durchgeführt. Erfindungsgemäß bevorzugt ist daher ein Salzgehalt von weniger als 0,1 Gewichts-% und ein Chlorgehalt von 0 mg/l.

Das erfindungsgemäße Verfahren ist erfindungsgemäß vorteilhaft dadurch gekennzeichnet, dass der mit der Schablone präparierte Unterarm in Verfahrensschritt d) unter der Wasseroberfläche liegt und mit einer Wassersäule von 1 bis 35 cm überdeckt ist. Dabei ist es erfindungsgemäß bevorzugt, wenn der präparierte Unterarm mit einer Wassersäule von 3 cm überdeckt ist. Da der Durchmesser des Unterarms des Menschen üblicherweise zum Ellenbogen hin ansteigt, wird als Bezugspunkt für die Wassersäulenhöhe mittig zwischen dem unbehandelten und dem mit Sonnenschutzmittel behandelten Areal gewählt. Geringe Schwankungen der Höhe der Wassersäule von bis zu 2 cm sind für die Messung in der Regel jedoch unkritisch, da die UV-Absorption des Wassers relativ gering ist.

Für das erfindungsgemäße Verfahren ist es erfindungsgemäß von Vorteil, wenn im Verfahrensschritt e) die Entfernung der UV-Lampe von der Wasseroberfläche von 10 bis 40 cm beträgt. Dabei ist eine Entfernung von 20 cm von der Wasseroberfläche erfindungsgemäß bevorzugt.

Für das erfindungsgemäße Verfahren ist es erfindungsgemäß vorteilhaft, wenn die UV-Lampe im Verfahrensschritt e) im Wellenlängenbereich von 290 nm bis 780 nm (UV und VIS) eine Gesamt-Strahlungsstärke von 150 W/m$^2$ bis 500 W/m$^2$ aufweist. Erfindungsgemäß bevorzugt wird ein Flächenstrahler, der eine homogene Bestrahlungsstärke über eine Mindestfläche von 20 cm x 20 cm aufweist.

Erfindungsgemäß bevorzugt wird eine Xenon-Gas-Lampe eingesetzt. Erfindungsgemäß bevorzugt ist dabei eine Xenon-Gas-Lampe mit einer Leistung von 1600 W. Die UV-Strahlung der Lampe sollte üblicherweise der ISO24444 entsprechen.

Für das erfindungsgemäße Verfahren ist es darüber hinaus vorteilhaft und empfehlenswert, wenn die Leistung der Lampe jeweils vor Beginn der Messung noch einmal mit Hilfe eines Radiometers (Beispielsweise IL1700, InternationalLight Technologies) bestimmt wird.

Als UV-Lampe (Strahlungsquelle) kann beispielsweise ein Oriel Sol 3A Solar Simulator der Firma Newport verwendet werden.

Es ist erfindungsgemäß vorteilhaft, wenn im Verfahrensschritt e) der Zeitraum der Bestrahlung $T_1$ von 2 bis 10 Minuten beträgt. Erfindungsgemäß bevorzugt beträgt die Bestrahlungszeit $T_1$ 6 Minuten.

Ferner ist es erfindungsgemäß von Vorteil, wenn nach der Bestrahlung eine jeweils eine gleich große Anzahl der Öffnungen in der Schablone, welche die mit dem Sonnenschutzmittel behandelten als auch die unbehandelten Hautareale bedecken, mit einem UV-Licht undurchlässigen Medium Licht-undurchlässig abgedeckt werden.

Es ist für das erfindungsgemäße Verfahren vorteilhaft, wenn im Verfahrensschritt f) die Öffnungen mit einem selbstklebenden, undurchsichtigen, wasserfesten Klebeband auf der dem Arm gegenüberliegenden Außenseite der Schablone abgeklebt werden. Beispielsweise kann hierfür Tesa Extra Power® verwendet werden. Die entsprechenden Öffnungen werden dann mit dem Klebeband einfach überklebt.

Es ist erfindungsgemäß vorteilhaft, wenn im Verfahrensschritt f) der Zeitraum $T_2$ und die folgenden Zeiträume $T_n$ so gewählt werden, dass sich die UV-Dosis prozentual um den gleichen Anteil erhöht verglichen mit Zeitraum $T_1$ bzw. $T_{n-1}$. Erfindungsgemäß bevorzugt werden 1,15-fache Inkremente.

Darüber hinaus ist es erfindungsgemäß bevorzugt, wenn im Verfahrensschritt g) die Teilmenge an zusätzlich abgedeckten Öffnungen gleich groß ist wie im Verfahrensschritt f) nach der Zeit $T_1$.

Nicht zuletzt ist es erfindungsgemäß bevorzugt, wenn die Anzahl der nach einem weiteren Bestrahlungszeitraum zusätzlich abgedeckten Öffnungen in der Schablone der Anzahl der in Verfahrensschritt f) abgedeckten Areale beträgt.

**Ausführungsbeispiel**

Das folgende Ausführungsbeispiel soll das erfindungsgemäße Verfahren erläutern ohne es einzuschränken: Nachdem der Proband über den Ablauf der Studie informiert wurde, dieser hinsichtlich der Einhaltung der Studienanforderungen überprüft wurde und er seine schriftliche Einwilligung zur Teilnahme an der Studie gegeben hat, wird auf der unteren Hälfte des rechten volaren Unterarms des Probanden ein Areal von 5 cm Breite und 7 cm Länge markiert. Auf einer Feinwaage (Mettler Toledo; XP205 DeltaRange®) werden 70 $\pm$ 1 mg des Sonnenschutzproduktes Nivea Sun ADULT SPRAY SPF 10 RL2016 abgewogen. Die 70 mg werden mit einem Fingerling (Latex REF 6091032; Braun) gleichmäßig auf das markierte Areal appliziert, so dass sich eine Auftragsmenge von 2 mg/cm$^2$ ergibt. Nach 15 Minuten wird auf dem Arm mit doppelseitigem Klebeband eine lichtundurchlässige Schablone aus Baumwollgewebe mit einer Acrylatbeschichtung appliziert. Die Schablone weist 12 kreisrunde Löcher mit einem Durchmesser von 1 cm auf. Die 12 Löcher sind auf 2 Gruppen a 6 Löcher aufgeteilt. Diese 6 Löcher wiederum sind in der Form 2 * 3 angeordnet jeweils mit einem Abstand von 4 mm zueinander. Die beiden 6er-Gruppen haben einen Abstand von 4 cm. Die Schablone wird so appliziert, dass sich sechs Löcher auf dem mit dem Lichtschutzmittel behandelten Areal und sechs Löcher auf einem benachbarten unbehandelten Areal befinden. Der abgeklebte Arm wird in einer Kunststoffbox mit den Maßen 530 mm * 378 mm und 200 mm Höhe so platziert, dass die zu bestrahlenden Areale waagerecht nach oben ausgerichtet sind. Die Kunststoffbox wird mit ca. 30 Liter Leitungswasser mit einer Temperatur von 29°C gefüllt. Die genaue Wassermenge wird so gewählt, dass die Wassersäule über dem Arm, gemessen mittig zwischen den beiden 6er-Gruppen Löchern, 3 cm beträgt. Beim Einfüllen des Wassers wird darauf geachtet, dass das Wasser nicht über dem Arm, insbesondere auf dem mit Lichtschutzmittel behandelten Areal, eingefüllt wird. Anschließend wird ein UV-Sonnensimulator in Form eines Flächenstrahlers (Oriel Sonnensimulator, Model 92294; LOT Oriel; gefiltert mit einem WG320-Filter) mittig über den beiden zu bestrahlenden Arealen in einem Abstand von 20 cm über der Wasseroberfläche platziert. Die Strahlungs-Leistung der UV-Quelle wurde direkt vorher mit Hilfe eines Radiometers (IL1700, InternationalLight Technologies) und einer UVB-Sonde (SED240, InternationalLight Technologies) im Abstand der Bestrahlung mit 0,245 mW/cm$^2$ bestimmt. Die individuelle MED (minimale Erythemdosis) des Probanden auf dem Unterarm ist aus vorangegangenen Bestrahlungen mit 130 mJ/cm$^2$ bekannt. Die Bestrahlungsdauer auf dem unbehandelten Areal wird so gewählt, dass die MED auf der vierten Bestrahlungs-Stufe erwartet wird. Das Increment für die einzelnen Bestrahlungsstufen wird auf 1,15 festgelegt. Für das unbehandelte Areal ergeben sich damit die folgenden Bestrahlungsdosen bzw. Bestrahlungszeiten, nach denen die einzelnen Areale abgeklebt werden:

*Tabelle 1: Bestrahlungsdosen und Bestrahlungszeiten für das unbehandelte Areal (Bestrahlungszeit [s] = Dosis [mJ/cm²] / Strahlungsleistung [mW1cm²] in diesem Fall mit Strahlungsleistung = 0,245 mW1cm²)*

|  | Bestrahlungszeit [min : sec] | Dosis [mJ/cm²] |
|---|---|---|
| 1. Stufe | 5: 48 | 85 |
| 2. Stufe | 6: 41 | 98 |
| 3. Stufe | 7: 41 | 113 |
| 4. Stufe | 8: 51 | 130 |
| 5. Stufe | 10: 10 | 150 |
| 6. Stufe | 11: 42 | 172 |

Für das Produkt wird unter Wasser ein SPF von ca. 7 erwartet. Mit diesem Faktor werden die Bestrahlungsdosen und Bestrahlungszeiten der 4. Stufe für das mit UV-Schutz behandelte Areal multipliziert (durch die Incrementierung ergeben sich teilweise leichte Rundungsdifferenzen für die anderen Bestrahlungsstufen):

*Tabelle 2: Bestrahlungsdosen und Bestrahlungszeiten für das mit Lichtschutz behandelte Areal*

|  | Bestrahlungszeit [min : sec] | Dosis [mJ/cm²] |
|---|---|---|
| 1. Stufe | 40: 42 | 598 |
| 2. Stufe | 46: 49 | 688 |
| 3. Stufe | 53: 50 | 791 |
| 4. Stufe | 61: 54 | 910 |
| 5. Stufe | 71: 11 | 1047 |
| 6. Stufe | 81: 52 | 1203 |

Die entsprechenden Areale werden nach den angegebenen Zeiten mit einem unter Wasser haftenden, lichtundurchlässigem Klebeband (Tesa Extra Power®) abgeklebt. 20 Stunden nach der Bestrahlung erscheint der Proband erneut. Zu diesem Zeitpunkt werden die bestrahlten Areale visuell von einer geschulten Person begutachtet. Auf beiden bestrahlten Arealen wird das Areal ermittelt, auf dem eine MED erkennbar ist. Eine MED ist die Dosis, bei der eine gerade eben wahrnehmbare Rötung mit gut abgrenzbaren Rändern zu erkennen ist. In diesem Fall erkennt man eine MED auf dem unbehandelten Areal bei der Dosis der Stufe 4 und auf dem mit Sonnenschutzmittel behandelten Areal ebenfalls auf dem Areal der Stufe 4.

Den individuellen UnterWasserSPF erhält man, wenn man die MED$_{geschützt}$ ins Verhältnis zur MED$_{ungeschützt}$ setzt. In diesem Fall ergibt sich:

$$SPF_{UW} = (910 \text{ mJ/cm}^2) / (130 \text{ mJ/cm}^2) = 7$$

Insgesamt wurden für das Produkt Messungen an 16 Probanden mit heller Haut (Phototyp I oder II) gemacht. In Tabelle 3 sind die Ergebnisse aller Probanden dargestellt. Ein Proband (lfd. Nr. 4) konnte nicht ausgewertet werden, da die Bestrahlungen nicht lange genug waren, um ausreichende Rötungen zu induzieren. Als Beispiel wurde die Messung des Probanden mit der laufenden Nummer 5 dargestellt.

Tabelle 3: Zusammenfassung aller „unter Wasser-Messungen" für das Produkt Nivea Sun ADULT SPRAY SPF 10 RL2016

| Teilnehmer der Studie | | | SPF | | | applizierte Menge [mg] | | Wasser tempera tur [°C] |
|---|---|---|---|---|---|---|---|---|
| No | Age | Sex | MEDu [mJ/cm²] | MEDp [mJ/cm²] | SPF$_{individual}$ | [mg] | [mg/cm²] | |
| 1 | 27 | w | 125 | 966 | **7.7** | 70.2 | 2.01 | 29.4 |
| 2 | 63 | w | 175 | 552 | **3.2** | 69.8 | 1.99 | 29.4 |
| 3 | 58 | m | 95 | 648 | **6.8** | 70.35 | 2.01 | 29.8 |
| 4 | 48 | m | - | - | | 70.2 | 2.01 | 29.9 |
| 5 | 45 | w | 130 | 910 | **7.0** | 70.38 | 2.01 | 28.5 |
| 6 | 60 | w | 200 | 1000 | **5.0** | 69.87 | 2.00 | 29.5 |
| 7 | 49 | w | 160 | 670 | **4.2** | 70.5 | 2.01 | 29.6 |
| 8 | 62 | w | 188 | 700 | **3.7** | 70.4 | 2.01 | 29.5 |
| 9 | 63 | w | 115 | 910 | **7.9** | 70.5 | 2.01 | 28.8 |
| 10 | 47 | w | 185 | 1185 | **6.4** | 69.9 | 2.00 | 28.9 |
| 11 | 44 | m | 132 | 900 | **6.8** | 69.81 | 1.99 | 29.7 |
| 12 | 52 | m | 130 | 1050 | **8.1** | 70.29 | 2.01 | 29.9 |
| 13 | 59 | w | 130 | 800 | **6.2** | 70.15 | 2.00 | 29.1 |
| 14 | 59 | w | 120 | 785 | **6.5** | 69.75 | 1.99 | 29.8 |
| 15 | 54 | m | 100 | 1100 | **11.0** | 70.3 | 2.01 | 29.9 |
| 16 | 50 | m | 122 | 780 | **6.4** | 69.95 | 2.00 | 30.4 |
| N | 16 | | 15 | 15 | 15 | | | |
| **mean** | **52.5** | | **140.5** | **863.7** | **6.5** | | | |
| Std.dev | 9.4 | | 32.9 | 180.0 | 2.0 | | | |
| Var.coeff. | 17.9% | | 23.4% | 20.8% | 30.2% | | | |
| CI(0.95) | | | | | 1.1 | | | |
| CI(0.95) [%] | | | | | 16.7% | | | |
| min | 27.0 | | 95.0 | 552.0 | 3.2 | | | |
| max | 63.0 | | 200.0 | 1185.0 | 11.0 | | | |
| median | 53.0 | | 130.0 | 900.0 | 6.5 | | | |

**Patentansprüche**

1. Verfahren zur Bestimmung des in-vivo UV-Lichtschutzes (SPF) von Sonnenschutzmitteln auf der Haut im Wasser, wobei

    a) ein definiertes Hautareal einer Testperson mit einer definierten Menge an zu testendem Sonnenschutzmittel bestrichen wird,

    b) man das Sonnenschutzmittel über einen definierten Zeitraum in die Haut einziehen lässt,

    c) nach dem Einziehen des Sonnenschutzmittels in die Haut, eine UV-Licht undurchlässige Schablone auf dem Hautareal aufgebracht wird, welche eine definierte Anzahl von Öffnungen definierter Größe aufweist, wobei eine bestimmte Anzahl der Öffnungen über den mit dem Sonnenschutzmittel behandelten Teil des Hautareals und eine weitere bestimmte Anzahl der Öffnungen über einen mit dem Sonnenschutzmittel unbehandelten Teil des Hautareals gelegt und fixiert wird,

    d) das so präparierte Hautareal in ein wasserdichtes Behältnis gelegt wird, welches mit Wasser befüllt ist, wobei das Hautareal unter der Wasseroberfläche liegt und mit einer definierten Höhe der Wassersäule überdeckt ist,

    e) oberhalb des Hautareals und der ihn bedeckenden Wassersäule in einer definierten Entfernung eine UV-Lampe definierter Strahlungsstärke angebracht und fixiert wird, mit welcher das präparierte Hautareal über einen definierten Zeitraum $T_1$ bestrahlt wird,

    f) nach Ablauf der Bestrahlungszeit $T_1$ eine Teilmenge der Öffnungen in der Schablone mit einem UV-Licht undurchlässigen Medium Licht-undurchlässig abgedeckt werden und das so präparierte Hautareal erneut einen weiteren Zeitraum $T_2$ unter den gleichen Bedingungen wie bei $T_1$ bestrahlt wird,

g) wobei nach Ablauf der Bestrahlungszeit $T_2$ eine weitere, zusätzliche Teilmenge der Öffnungen in der Schablone mit einem UV-Licht undurchlässigen Medium Licht-undurchlässig abgedeckt werden und das so präparierte Hautareal erneut einen weiteren Zeitraum $T_3$ unter den gleichen Bedingungen wie bei $T_1$ bestrahlt wird,

h) die Verfahrensschritte f) und g) maximal solange wiederholt werden, bis alle Öffnungen der Schablone verschlossen sind, wobei die Öffnungen der Schablone, die die mit Sonnenschutzmittel behandelten Areale betreffen, entsprechend des erwarteten Schutzfaktors später abgedeckt als die Öffnungen der Schablone, die die unbehandelten Areale betreffen,

i) anschließend das präparierte Hautareal aus dem Behältnis entnommen, die Schablone samt den Lochabdeckungen vom Hautareal entfernt wird,

j) am nächsten Tag d.h. nach 20 $\pm$ 4 h die minimale Erythemdosis MED der mit dem Sonnenschutzmittel behandelten ($MED_{Sonnenschutzmittel}$) und unbehandelten Hautareale ($MED_{unbehandelte\ Haut}$) anhand der aufgetretenen Hautrötungen und den entsprechenden Bestrahlungsdosen, die sich aus den unterschiedlichen Bestrahlungszeiten ergeben, bestimmt wird und

k) aus beiden Werten nach der Formel SPF= $MED_{Sonnenschutzmittel}$/ $MED_{unbehandelte\ Haut}$ der Unterwasser-Lichtschutzfaktor des Sonnenschutzmittels errechnet wird, oder, bei höheren Lichtschutzfaktoren, bei denen auf dem mit Sonnenschutzmittel behandelten Areal im Rahmen der Bestrahlungszeit keine MED mehr erreicht wird, eine Mindestschutzzeit unter Wasser entsprechend der maximalen Bestrahlungsdauer ermittelt wird.

2. Verfahren nach Anspruch 1, **dadurch gekennzeichnet, dass** als Hautareal ein Areal des Unterarms ausgewählt wird.

3. Verfahren nach einem der vorhergehenden Ansprüche, **dadurch gekennzeichnet, dass** im Verfahrensschritt a) das definierte Hautareal als Teil des Unterarms einer Testperson, welches mit einer definierten Menge an zu testendem Sonnenschutzmittel bestrichen wird, die Größe von 12 cm$^2$ bis 64 cm$^2$ aufweist.

4. Verfahren nach einem der vorhergehenden Ansprüche, **dadurch gekennzeichnet, dass** man im Verfahrensschritt a) das Sonnenschutzmittel mit Hilfe eines Fingerlings auf die Haut aufträgt.

5. Verfahren nach einem der vorhergehenden Ansprüche, **dadurch gekennzeichnet, dass** man im Verfahrensschritt a) das Sonnenschutzmittel in einer Menge von 2 mg/cm$^2$ auf die Haut aufträgt.

6. Verfahren nach einem der vorhergehenden Ansprüche, **dadurch gekennzeichnet, dass** man im Verfahrensschritt b) das Sonnenschutzmittel über einen Zeitraum von 15 Minuten in die Haut einziehen lässt.

7. Verfahren nach einem der vorhergehenden Ansprüche, **dadurch gekennzeichnet**, die UV-Licht undurchlässige Schablone, die in Verfahrensschritt c) auf den Unterarm aufgebracht wird, eine den Unterarm umschließende Arm-Manschette ist.

8. Verfahren nach einem der vorhergehenden Ansprüche, **dadurch gekennzeichnet**, die UV-Licht undurchlässige Schablone, die in Verfahrensschritt c) auf den Unterarm aufgebracht wird, aus Baumwollgewebe mit einer Acrylatbeschichtung besteht.

9. Verfahren nach einem der vorhergehenden Ansprüche, **dadurch gekennzeichnet, dass** die UV-Licht undurchlässige Schablone, die in Verfahrensschritt c) auf den Unterarm aufgebracht wird, 8-20 Öffnungen definierter Größe aufweist.

10. Verfahren nach einem der vorhergehenden Ansprüche, **dadurch gekennzeichnet, dass** die UV-Licht undurchlässige Schablone, die in Verfahrensschritt c) auf den Unterarm aufgebracht wird, kreisförmige Öffnungen aufweist.

11. Verfahren nach einem der vorhergehenden Ansprüche, **dadurch gekennzeichnet, dass** die UV-Licht undurchlässige Schablone, die in Verfahrensschritt c) auf den Unterarm aufgebracht wird, kreisförmige Öffnungen mit einem Durchmesser von jeweils 1 cm aufweist.

12. Verfahren nach einem der vorhergehenden Ansprüche, **dadurch gekennzeichnet, dass** die UV-Licht undurchlässige Schablone, die in Verfahrensschritt c) auf den Unterarm aufgebracht wird, so fixiert wird, dass jeweils die gleiche Anzahl der Öffnungen über den mit dem Sonnenschutzmittel behandelten Teil des Unterarms und dem mit dem Sonnenschutzmittel unbehandelten Teil des Unterarms liegt.

**13.** Verfahren nach einem der vorhergehenden Ansprüche, **dadurch gekennzeichnet, dass** der mit der Schablone präparierte Unterarm in Verfahrensschritt d) in ein wasserdichtes Behältnis gelegt wird, welches eine Länge von 530mm, eine Breite von 375mm und eine Höhe von 200mm aufweist.

**14.** Verfahren nach einem der vorhergehenden Ansprüche, **dadurch gekennzeichnet, dass** der mit der Schablone präparierte Unterarm in Verfahrensschritt d) in ein wasserdichtes Behältnis gelegt wird, das aus Kunststoff besteht.

**15.** Verfahren nach einem der vorhergehenden Ansprüche, **dadurch gekennzeichnet, dass** der mit der Schablone präparierte Unterarm in Verfahrensschritt d) in ein wasserdichtes Behältnis gelegt wird welches mit Wasser befüllt ist, das während der Verfahrensdurchführung eine konstante Temperatur aufweist.

**16.** Verfahren nach einem der vorhergehenden Ansprüche, **dadurch gekennzeichnet, dass** der mit der Schablone präparierte Unterarm in Verfahrensschritt d) in ein wasserdichtes Behältnis gelegt wird, welches mit Wasser befüllt ist, das während der Verfahrensdurchführung eine Temperatur von 15 bis 35 °C aufweist.

**17.** Verfahren nach einem der vorhergehenden Ansprüche, **dadurch gekennzeichnet, dass** der mit der Schablone präparierte Unterarm in Verfahrensschritt d) in ein wasserdichtes Behältnis gelegt wird welches mit Wasser befüllt ist, das einen Salzgehalt von 0 bis 30 Gewichts-% aufweist.

**18.** Verfahren nach einem der vorhergehenden Ansprüche, **dadurch gekennzeichnet, dass** der mit der Schablone präparierte Unterarm in Verfahrensschritt d) in ein wasserdichtes Behältnis gelegt wird, welches mit Wasser befüllt ist, das einen Chlorgehalt von 0 bis 3 mg/l aufweist.

**19.** Verfahren nach einem der vorhergehenden Ansprüche, **dadurch gekennzeichnet, dass** der mit der Schablone präparierte Unterarm in Verfahrensschritt d) unter der Wasseroberfläche liegt und mit einer Wassersäule von 1 bis 35 cm überdeckt ist.

**20.** Verfahren nach einem der vorhergehenden Ansprüche, **dadurch gekennzeichnet, dass** im Verfahrensschritt e) die Entfernung der UV-Lampe von der Wasseroberfläche von 10 bis 40 cm beträgt.

**21.** Verfahren nach einem der vorhergehenden Ansprüche, **dadurch gekennzeichnet, dass** die UV-Lampe im Verfahrensschritt e) im Wellenlängenbereich von 290 nm bis 780 nm eine Gesamt-Strahlungsstärke von 150 W/m$^2$ bis 500 W/m$^2$ aufweist.

**22.** Verfahren nach einem der vorhergehenden Ansprüche, **dadurch gekennzeichnet, dass** im Verfahrensschritt e) der Zeitraum der Bestrahlung $T_1$ von 2 bis 10 Minuten beträgt.

**23.** Verfahren nach einem der vorhergehenden Ansprüche, **dadurch gekennzeichnet, dass** nach der Bestrahlung jeweils eine gleich große Anzahl der Öffnungen in der Schablone, welche die mit dem Sonnenschutzmittel behandelten als auch die unbehandelten Hautareale bedecken, mit einem UV-Licht undurchlässigen Medium Licht-undurchlässig abgedeckt werden.

**24.** Verfahren nach einem der vorhergehenden Ansprüche, **dadurch gekennzeichnet, dass** im Verfahrensschritt f) die Öffnungen mit einem selbstklebenden, undurchsichtigen, wasserfesten Klebeband auf der dem Arm gegenüberliegenden Außenseite der Schablone abgeklebt werden.

**25.** Verfahren nach einem der vorhergehenden Ansprüche, **dadurch gekennzeichnet, dass** im Verfahrensschritt f) der Zeitraum $T_2$ und die folgenden Zeiträume $T_n$ so gewählt werden, dass sich die UV-Dosis prozentual um den gleichen Anteil erhöht verglichen mit Zeitraum $T_1$ bzw. $T_{n-1}$.

**26.** Verfahren nach einem der vorhergehenden Ansprüche, **dadurch gekennzeichnet, dass** im Verfahrensschritt g) die Teilmenge an zusätzlich abgedeckten Öffnungen gleich groß ist wie im Verfahrensschritt f) nach der Zeit $T_1$.

**27.** Verfahren nach einem der vorhergehenden Ansprüche, **dadurch gekennzeichnet, dass** die Anzahl der nach einem weiteren Bestrahlungszeitraum zusätzlich abgedeckten Öffnungen in der Schablone der Anzahl der in Verfahrensschritt f) abgedeckten Areale beträgt.

**Claims**

1. Method for determining the *in vivo* UV light protection (SPF) afforded by sunscreens on the skin in the water, where

   a) a defined area of the skin of a test person is coated with a defined amount of sunscreen under test,
   b) the skin is allowed to draw in the sunscreen over a defined period,
   c) after the drawing of the sunscreen into the skin, a stencil impervious to UV light is applied to the area of skin, the stencil having a defined number of openings of defined size, with a certain number of the openings being placed and fixed over the part of the area of skin treated with the sunscreen, and a further certain number of the openings being placed and fixed over a part of the area of skin untreated with the sunscreen,
   d) the area of skin thus prepared is placed into a watertight container filled with water, the area of skin lying below the water surface and being covered by a defined height of the water column,
   e) above the area of skin and the water column covering it, at a defined distance, a UV lamp of defined radiant intensity is mounted and fixed, and is used to irradiate the prepared area of skin over a defined period $T_1$,
   f) after the irradiation time $T_1$ has elapsed, a subset of the openings in the stencil are masked light-impermeably with a medium impervious to UV light, and the area of skin thus prepared is again irradiated for a further period $T_2$ under the same conditions as for $T_1$,
   g) wherein, after the irradiation time $T_2$ has elapsed, a further, additional subset of the openings in the stencil are covered light-impermeably with a medium impervious to UV light, and the area of skin thus prepared is again irradiated for a further period $T_3$ under the same conditions as for $T_1$,
   h) method steps f) and g) are repeated for at most until all of the openings in the stencil have been obturated, with the openings of the stencil relating to the areas treated with sunscreen being covered later, in accordance with the anticipated protection factor, than the openings in the stencil which relate to the untreated areas,
   i) subsequently the prepared area of skin is withdrawn from the container, and the stencil together with the hole coverings is removed from the area of skin,
   j) on the next day, i.e. after 20 $\pm$ 4h, the minimum erythemal dose MED of the areas of skin treated (MED $_{sunscreen}$) and untreated (MED $_{untreated\ skin}$) with sunscreen is determined on the basis of the skin reddening that has occurred and of the corresponding irradiation doses resulting from the different irradiation times, and
   k) the underwater sun protection factor of the sunscreen is computed from the two values according to the formula SPF = MED $_{sunscreen}$/MED $_{untreated\ skin}$, or, in the case of higher sun protection factors, for which an MED is no longer achieved on the area treated with sunscreen within the irradiation time, a minimum protection time under water is determined, corresponding to the maximum irradiation duration.

2. Method according to Claim 1, **characterized in that** an area of the forearm is selected as area of skin.

3. Method according to either of the preceding claims, **characterized in that** in method step a) the defined area of skin, as part of the forearm of a test person, which is coated with a defined amount of sunscreen under test, has a size of 12 cm$^2$ to 64 cm$^2$.

4. Method according to any of the preceding claims, **characterized in that** in method step a) the sunscreen is applied to the skin with the aid of a fingerstall.

5. Method according to any of the preceding claims, **characterized in that** in method step a) the sunscreen is applied to the skin in an amount of 2 mg/cm$^2$.

6. Method according to any of the preceding claims, **characterized in that** in method step b) the skin is allowed to take in the sunscreen over a period of 15 minutes.

7. Method according to any of the preceding claims, **characterized in that** the stencil impervious to UV light that is applied to the forearm in method step c) is an arm sleeve surrounding the forearm.

8. Method according to any of the preceding claims, **characterized in that** the stencil impervious to UV light that is applied to the forearm in method step c) consists of cotton fabric with an acrylate coating.

9. Method according to any of the preceding claims, **characterized in that** the stencil impervious to UV light that is applied to the forearm in method step c) has 8-20 openings of defined size.

10. Method according to any of the preceding claims, **characterized in that** the stencil impervious to UV light that is applied to the forearm in method step c) has circular openings.

11. Method according to any of the preceding claims, **characterized in that** the stencil impervious to UV light that is applied to the forearm in method step c) has circular openings having a diameter of in each case 1 cm.

12. Method according to any of the preceding claims, **characterized in that** the stencil impervious to UV light which is applied to the forearm in method step c) is fixed in such a way that in each case the number of openings over the part of the forearm treated with the sunscreen is the same as the number of openings over the part of the forearm not treated with the sunscreen.

13. Method according to any of the preceding claims, **characterized in that** the forearm prepared with the stencil is placed in method step d) into a watertight container which has a length of 530 mm, a width of 375 mm and a height of 200 mm.

14. Method according to any of the preceding claims, **characterized in that** the forearm prepared with the stencil is placed in method step d) into a watertight container which consists of plastic.

15. Method according to any of the preceding claims, **characterized in that** the forearm prepared with the stencil is placed in method step d) into a watertight container which is filled with water that has a constant temperature during the implementation of the method.

16. Method according to any of the preceding claims, **characterized in that** the forearm prepared with the stencil is placed in method step d) into a watertight container which is filled with water which has a temperature of 15 to 35°C during the implementation of the method.

17. Method according to any of the preceding claims, **characterized in that** the forearm prepared with the stencil is placed in method step d) into a watertight container which is filled with water which has a salt content of 0 to 30 weight%.

18. Method according to any of the preceding claims, **characterized in that** the forearm prepared with the stencil is placed in method step d) into a watertight container which is filled with water which has a chlorine content of 0 to 3 mg/l.

19. Method according to any of the preceding claims, **characterized in that** the forearm prepared with the stencil lies under the water surface in method step d) and is covered with a water column of 1 to 35 cm.

20. Method according to any of the preceding claims, **characterized in that** in method step e) the distance of the UV lamp from the water surface is from 10 to 40 cm.

21. Method according to any of the preceding claims, **characterized in that** the UV lamp in method step e) has a total radiant intensity in the wavelength range from 290 nm to 780 nm of 150 $W/m^2$ to 500 $W/m^2$.

22. Method according to any of the preceding claims, **characterized in that** in method step e) the period of irradiation $T_1$ is from 2 to 10 minutes.

23. Method according to any of the preceding claims, **characterized in that** after the irradiation, in each case an equal number of the openings in the stencil that cover the areas of skin treated with the sunscreen and the untreated areas of skin are masked light-impermeably with a medium impervious to UV light.

24. Method according to any of the preceding claims, **characterized in that** in method step f) the openings are taped off with a self-adhesive, non-transparent, water-resistant adhesive tape on the outside of the stencil opposite the arm.

25. Method according to any of the preceding claims, **characterized in that** in method step f) the period $T_2$ and the following periods Tn are selected such that the UV dose is increased by the same proportion in percentage terms as compared with period $T_1$ and $T_{n-1}$, respectively.

26. Method according to any of the preceding claims, **characterized in that** in method step g) the subset of additionally masked openings is the same size as in method step f) after the time $T_1$.

**27.** Method according to any of the preceding claims, **characterized in that** the number of openings in the stencil that are additionally masked after a further irradiation period amounts to the number of the areas masked in method step f).

## Revendications

**1.** Procédé de détermination de la protection contre la lumière UV (SPF) in vivo d'agents de protection solaire sur la peau dans l'eau, procédé selon lequel

a) une zone cutanée définie d'une personne testée est enduite d'une quantité définie de moyens de protection solaire à tester,

b) on laisse l'agent de protection solaire pénétrer dans la peau pendant une durée définie,

c) après que l'agent de protection solaire a pénétré dans la peau, un cache imperméable à la lumière UV est appliqué sur la zone de la peau, lequel comporte un nombre défini d'ouvertures d'une taille définie, un certain nombre des ouvertures étant placées et fixées sur la partie de la zone cutanée qui a été traitée avec l'agent de protection solaire et un autre nombre défini des ouvertures étant placées et fixées sur une partie de la zone cutanée qui n'a été pas traitée avec l'agent de protection solaire,

d) la zone cutanée ainsi préparée est placée dans un récipient étanche à l'eau et rempli d'eau, la zone cutanée étant placée sous la surface de l'eau et recouverte d'une hauteur définie de la colonne d'eau,

e) une lampe UV d'intensité de rayonnement définie est montée et fixée à une distance définie au-dessus de la zone cutanée et de la colonne d'eau qui la recouvre et est utilisée pour irradier la zone cutanée préparée pendant une durée définie $T_1$,

f) au bout du temps d'irradiation $T_1$, certaines des ouvertures du cache sont recouvertes d'un milieu imperméable aux UV et la zone cutanée ainsi préparée est à nouveau irradiée pendant une nouvelle durée $T_2$ dans les mêmes conditions que pour $T_1$,

g) au bout du temps d'irradiation $T_2$, un autre sous-ensemble supplémentaire d'ouvertures ménagées dans le cache est recouvert d'un milieu imperméable aux UV et la zone cutanée ainsi préparée est à nouveau irradiée pendant une durée supplémentaire $T_3$ dans les mêmes conditions que pour $T_1$,

h) les étapes du procédé f) et g) sont répétées au maximum jusqu'à ce que toutes les ouvertures du cache soient obturées, les ouvertures du cache qui concernent les zones traitées avec l'agent de protection solaire étant recouvertes selon le facteur de protection attendu plus tard que les ouvertures du cache qui concernent les zones non traitées,

i) ensuite la zone cutanée préparée est retirée du récipient, le cache étant retiré de la zone de peau conjointement avec le revêtement des trous,

j) le lendemain, c'est-à-dire 20 ± 4 h après, la dose d'érythème minimale MED des zones cutanées traitées avec l'agent de protection solaire ($MED_{\text{agent de protection solaire}}$) et non traitées ($MED_{\text{peau non traitée}}$) est déterminée sur la base de la rougeur cutanée survenue et des doses de rayonnement correspondantes qui résultent des différents temps d'irradiation et

k) le facteur de protection solaire sous l'eau de l'agent de protection solaire est calculé à partir des deux valeurs selon la formule SPF = $MED_{\text{agent de protection solaire}}/MED_{\text{peau non traitée}}$, ou, dans le cas de facteurs de protection solaire plus élevés, pour lesquels plus aucune MED n'est atteinte sur la zone traitée avec un agent de protection solaire pendant le temps d'irradiation, un temps de protection minimum sous l'eau est déterminé en fonction du temps d'irradiation maximum.

**2.** Procédé selon la revendication 1, **caractérisé en ce qu'**une zone de l'avant-bras est choisie comme zone cutanée.

**3.** Procédé selon l'une des revendications précédentes, **caractérisé en ce que**, à l'étape de procédé a), la zone cutanée définie comme partie de l'avant-bras d'une personne testée, qui est enduite d'une quantité définie d'agent de protection solaire à tester, a une taille de 12 cm$^2$ à 64 cm$^2$.

**4.** Procédé selon l'une des revendications précédentes, **caractérisé en ce que**, à l'étape de procédé a), l'agent de protection solaire est appliqué sur la peau à l'aide d'un doigtier.

**5.** Procédé selon l'une des revendications précédentes, **caractérisé en ce que**, à l'étape de procédé a), l'agent de protection solaire est appliqué sur la peau à raison de 2 mg/cm$^2$.

**6.** Procédé selon l'une des revendications précédentes, **caractérisé en ce que**, à l'étape de procédé b), on laisse la peau absorber l'agent de protection solaire pendant une durée de 15 minutes.

7. Procédé selon l'une des revendications précédentes, **caractérisé en ce que** le cache imperméable à la lumière UV, qui est appliqué sur l'avant-bras à l'étape de procédé c), est un brassard entourant l'avant-bras.

8. Procédé selon l'une des revendications précédentes, **caractérisé en ce que** le cache imperméable à la lumière UV, qui est appliqué sur l'avant-bras à l'étape de procédé c), est en tissu de coton pourvu d'un revêtement acrylate.

9. Procédé selon l'une des revendications précédentes, **caractérisé en ce que** le cache imperméable à la lumière UV, qui est appliqué sur l'avant-bras à l'étape de procédé c), comporte 8 à 20 ouvertures de taille définie.

10. Procédé selon l'une des revendications précédentes, **caractérisé en ce que** le cache imperméable à la lumière UV, qui est appliqué sur l'avant-bras à l'étape de procédé c), comporte des ouvertures circulaires.

11. Procédé selon l'une des revendications précédentes, **caractérisé en ce que** le cache imperméable à la lumière UV, qui est appliqué sur l'avant-bras à l'étape de procédé c), comporte des ouvertures circulaires d'un diamètre de 1 cm chacune.

12. Procédé selon l'une des revendications précédentes, **caractérisé en ce que** le cache imperméable à la lumière UV, qui est appliqué sur l'avant-bras à l'étape de procédé c), est fixé de manière à ce que la partie de l'avant-bras qui a été traitée avec l'agent de protection solaire et la partie de l'avant-bras qui n'a pas été traitée avec l'agent de protection solaire comportent le même nombre d'ouvertures.

13. Procédé selon l'une des revendications précédentes, **caractérisé en ce que** l'avant-bras préparé avec le cache est placé à l'étape de procédé d) dans un récipient étanche à l'eau qui a une longueur de 530 mm, une largeur de 375 mm et une hauteur de 200 mm.

14. Procédé selon l'une des revendications précédentes, **caractérisé en ce que** l'avant-bras préparé avec le cache est placé à l'étape de procédé d) dans un récipient étanche à l'eau qui est en matière synthétique.

15. Procédé selon l'une des revendications précédentes, **caractérisé en ce que** l'avant-bras préparé avec le cache est placé à l'étape de procédé d) dans un récipient étanche à l'eau qui est rempli avec de l'eau qui est à une température constante pendant la mise en œuvre du procédé.

16. Procédé selon l'une des revendications précédentes, **caractérisé en ce que** l'avant-bras préparé avec le cache est placé à l'étape de procédé d) dans un récipient étanche à l'eau qui est rempli avec de l'eau qui est à une température de 15 à 35 °C pendant la mise en œuvre du procédé.

17. Procédé selon l'une des revendications précédentes, **caractérisé en ce que** l'avant-bras préparé avec le cache est placé à l'étape de procédé d) dans un récipient étanche à l'eau qui est rempli avec de l'eau qui a une teneur en sel de 0 à 30 % en poids.

18. Procédé selon l'une des revendications précédentes, **caractérisé en ce que** l'avant-bras préparé avec le cache est placé à l'étape de procédé d) dans un récipient étanche à l'eau qui est rempli avec de l'eau qui a une teneur en chlore de 0 à 3 mg/l.

19. Procédé selon l'une des revendications précédentes, **caractérisé en ce que** l'avant-bras préparé avec le cache est situé à l'étape de procédé d) sous la surface de l'eau et est recouvert d'une colonne d'eau de 1 à 35 cm.

20. Procédé selon l'une des revendications précédentes, **caractérisé en ce que**, à l'étape de procédé e), la distance de la lampe UV à la surface de l'eau est de 10 à 40 cm.

21. Procédé selon l'une des revendications précédentes, **caractérisé en ce que**, à l'étape de procédé e), la lampe UV a une intensité de rayonnement totale de 150 W/m$^2$ à 500 W/m$^2$ dans la gamme de longueurs d'onde de 290 nm à 780 nm.

22. Procédé selon l'une des revendications précédentes, **caractérisé en ce que**, à l'étape de procédé e), la durée d'irradiation $T_1$ est de 2 à 10 minutes.

23. Procédé selon l'une des revendications précédentes, **caractérisé en ce que**, après l'irradiation, un nombre égal d'ouvertures, ménagées dans le cache, qui recouvrent les zones cutanées traitées avec l'agent de protection solaire ainsi que les zones cutanées non traitées, sont recouvertes avec un milieu imperméable à la lumière UV de manière imperméable à la lumière.

24. Procédé selon l'une des revendications précédentes, **caractérisé en ce que**, à l'étape de procédé f), les ouvertures sont recouvertes d'une bande auto-adhésive, opaque et étanche à l'eau du côté extérieur du cache qui est opposé au bras.

25. Procédé selon l'une des revendications précédentes, **caractérisé en ce que**, à l'étape de procédé f), la durée $T_2$ et les durées suivantes $T_n$ sont choisies de telle sorte que la dose UV augmente en pourcentage dans la même proportion par rapport à la durée $T_1$ respectivement $T_{n-1}$.

26. Procédé selon l'une des revendications précédentes, **caractérisé en ce que**, à l'étape de procédé g), le sous-ensemble d'ouvertures supplémentaires recouvertes est le même qu'à l'étape de procédé f) au bout du temps $T_1$.

27. Procédé selon l'une des revendications précédentes, **caractérisé en ce que** le nombre d'ouvertures supplémentaires, ménagées dans le cache, qui sont recouvertes après une durée d'irradiation supplémentaire est égal au nombre de zones recouvertes à l'étape de procédé f).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10214054 A1

- WO 9855086 A1

**In der Beschreibung aufgeführte Nicht-Patentliteratur**

- Sunscreens' photochemical behaviour: in vivo evaluation by the stripping method. **MARGINEAN LAZAR et al.** International Journal of Cosmetic Science. Kluwer Academic Publishers, 01. Januar 1997, vol. 19, 87-101

- Protective and sunscreen emulsions containing a perfluoropolyether amide. **PANTINI et al.** SOFW-JOURNAL, SEIFEN, OELE, FETTE, WACHSE. VERLAG FUR CHEMISCHE INDUS-TRIE, 01. Mai 2001, vol. 127, 13-17